# EUROPEAN PATENT APPLICATION

(11) **EP 1 481 681 A1**
(43) Date of publication of application: **01.12.2004**
(21) Application number: 03425349.2
(22) Date of filing: 30.05.2003
(51) Int. Cl.: A61K 35/74, A23L 1/03, C12R 1/225, C12R 1/23, C12R 1/24, C12R 1/245, C12R 1/25

(54) **Lactic acid bacteria combination and compositions thereof**

(71) Applicant: De Simone, Claudio, 00040 Ardea (Roma) (IT)
(72) Inventor: De Simone, Claudio, 00040 Ardea (Roma) (IT)
(74) Representative: Spadaro, Marco

(57) **Abstract**

The present invention provides prophylaxis and treatment of dismicrobial conditions by means of combinations of highly concentrated lactic acid bacteria, compositions containing them suitable for administration to mammals, their use as medicaments or food supplements, in particular suitable for the prevention and/or treatment of a number of diseases, in particular imbalances of the natural flora of mammals.

According to the general concept of the present invention, the lactic acid bacteria combination consists of:
(a) from about 5% to about 95% by weight of total combination of *Lactobacillus pentosus* and
(b) from about 5% to about 95% by weight of total combination of at least one strain of *Streptococcus thermophilus* and/or *Lactobacillus paracasei* and/or *Lactobacillus helveticus;* and/or mutants or derivatives of (a) and/or (b).

Examples of diseases or disturbances to be treated are imbalances of bacteria flora, inflammatory condition or a degenerative disease of the gastrointestinal tract, liver, pancreas, vagina, mouth, anus or any fistolous tract.

## Description

This invention relates to combinations of lactic acid bacteria, compositions containing them suitable for administration to mammals, their use as medicaments or food supplements, in particular suitable for the prevention and/or treatment of a number of diseases, in particular, but not limited to, imbalances of the natural flora of mammals.

### Background of the Invention

In the past a wide variety of products intended to correct imbalances and/or restore bacteria flora to the gut have been available, for example to prevent or treat irritable bowel syndrome (IBS).

The role and composition of the intestinal bacteria flora and its relationship with the mucosa itself has been studied. It has been determined that, in the terminal section of the small intestine and of the colon, there exists a large concentration of bacteria which are in close and intimate contact with the covering surface of the intestine. In normal circumstances, there is a balance between the host populations and the various defense structures of the mucosa; these bacteria are indeed useful to eliminate toxic substances, produce substances absolutely necessary to the integrity of the organism as well as increase the defense capacities of the mucosa thanks mostly to the production of immunoglobulins and mucus. This balance is preserved because the "friendly" bacteria with beneficial and protective action, prevail over toxic bacteria. Any condition altering this balance (e.g. an infectious state, abuse of some drugs, most probably an inadequate diet) can boost the growth of aggressive bacteria which damage the defenses of the intestinal mucosa. This may lead to ulcers and lesions of the intestine which are the basis of these intestinal diseases and provoke some well-know symptoms such as diarrhoea, blood in the stools, weight loss, etc.

An alteration in the patrimony of the intestinal bacterial flora, and in particular the prevalence of aggressive bacteria, is at the origin of some of these symptoms.

This beneficial role of lactic acid bacteria is also known in other districts of the mammal organism. For instance, lactic acid bacteria exert also a beneficial role in maintaining the correct environment in the vagina, as well as in the mouth, and in general in the whole enteric tract.

The present invention provides prophylaxis and treatment of dismicrobial conditions by administration of a probiotic preparation characterized by a high concentration of protective lactic acid bacteria (for example 100 billion per gram). Daily administration of the preparation prevents the onset or correct the existing dismicrobial imbalance.

### Description of the Invention

An object of the present invention is a lactic acid bacteria combination consisting of:
(a) from about 5% to about 95% by weight of total combination of *Lactobacillus pentosus* and
(b) from about 5% to about 95% by weight of total combination of at least one strain of *Streptococcus thermophilus* and/or *Lactobacillus paracasei* and/or *Lactobacillus helveticus;* and/or mutants or derivatives of (a) and/or (b).

The lactic acid bacteria of the above combination are of the strains *Lactobacillus pentosus, Streptococcus thermophilus, Lactobacillus paracasei* and *Lactobacillus helveticus.* The present invention can be carried out also with mutants or derivatives of said strains, provided that they have the same or better activity.

Preferably, lactic acid bacteria of the above strains having the following 16S-23S region genetic sequences are used.

### Lactobacillus pentosus

CGAAATCGCTAGTAATCGCGGATCAGCATGCCGCGGTGAATACGTTC
CCGGGCCTTGTACACACCGCCCGTCACACCATGAGAGTTTGTAACAC
CCAAAGTCGGTGGGGTAACCTTTTAGGAACCAGCCGCCTAAGGTGG
GACAGATGATTAGGGTGAAGTCGTAACAAGGTAGCCGTAGGAGAAC
CTGCGGCTGGATCACCTCCTTTCTAAGGAATATTACGGAAACCTACA
CACGCGTCGAAACTTTGTTTAGTTTTGAGAGATTTAACTCTCAAAACT
TGTTCTTTGAAAACTAGATAATATCAAATATATTTTTCATAATGAAA
CCGAGAACACCGCGTTTTTTGAGTTTTTATTGAAGTTTAATTATCGC
TAAACTCATTAATCGCATTTACCGTTAGGTAAATGAGGTTAAGTTAAC
AAGGGCGCATGGTGAATGCCTTGGCACTAGGAGCCGATGAAGGACG
GGACTAACACCGATATGCTTCGGGGAGCTGTACGTAAGCTATGATCC
GGAGATTTCCGAATGGGGGG (SEQ ID NO: 1)

### Streptococcus thermophilus

TGTCGGAATCGTTAGTAATCGCGGATCAGCACGCCGCGGTGAATACG
TTCCCGGGCCTTGTACACACCGCCCGTCACACCACGAGAGTTTGTAA
CACCCGAAGTCGGTGAGGTAACCTTTTGGAGCCAGCCGCCTAAGGTG
GGACAGATGATTGGGGTGAAGTCGTAACAAGGTAGCCGTATCGGAA
GGTGCGGCTGGATCACCTCCTTTCTAAGGAAAAACGGAATGTACTTG
AGTTTCTTATTTAGTTTTGAGAGGTCTTGTGGGGCCTTAGCTCAGCT
GGGAGAGCGCCTGCTTTGCACGCAGGAGGTCAGCGGTTCGATCCCG
CTAGGCTCCATTGAATCGAAAGATTCAAGTATTGTCCATTGAAAATT
GAATATCTATATCAAATTCCATATGTAAGTAATTACATATAGATAGTA
ACAAGAAAATAAACCGAAACGCTGTGAATATTTAATGAGTTAGGTCG
AAAGGCCAAAATAAGGTTAAGTTAATAAGGGCGCACGGTGGATGCCT
TGGCACTAGAAGCCGATGAAGGACGTGACTAACGACGAAATGCTTTG

NGGGAGCTGTAAGTGAGCAATGATCCAAANATGTCCGAATGGGGGG
GGGAC (SEQ ID NO: 2)

### Lactobacillus paracasei

AGNACGCCGCGGTGAATACGTTCCCGGGCCTTGTACACACCGCCCGT
CACACCATGAGAGTTTGTAACACCCGAAGCCGGTGGCGTAACCCTTT
TAGGGAGCGAGCCGTCTAAGGTGGGACAAATGATTAGGGTGAAGTC
GTAACAAGGTAGCCGTAGGAGAACCTGCGGCTGGATCACCTTCTTTT
TAAAGGAAACAGACTGAAAGTCTGACGGAAACCTGCACACACGAAAC
TTTGTTTAGTTTTGAGGGGATCACCCTCAAGCACCCTAACGGGTGCG
ACTTTGTTCTTTGAAAACTGGATATCATTGTATTAATTGTTTTAAATT
GCCGAGAACACAGCGTATTTGTATGAGTTTCTGAAAAAGAAATTCGC
ATCGCATAACCGCTGACGCAAGTCAGTACAGGTTAAGTTACAAA
GGGCGCACGGTGGATGCCTTGGCACTAGGAGCCGATGAAGGACGGA
ACTAATACCGATATGCTTCGGGGAGCTATAAGTAAGCTTTGATCCGG
AGATTTCCCAATGGGGGGGAC (SEQ ID NO: 3)

### Lactobacillus helveticus

TCGCTAGTAATCGCGGTCAGAACGCCGCGGTGAATACGTTCCCGGGC
CTTGTACACACCGCCCGTCACACCATGGAAGTCTGCAATGCCCAAAG
CCGGTGGCCTAACCTTCGGGAAGGAGCCGTCTAAGGCAGGGCAGAT
GACTGGGGTGAAGTCGTAACAAGGTAGCCGTAGGAGAACCTGCGGC
TGGATCACCTCCTTTCTAAGGAAGCTGAAGTGATGGAGAGTAGAGAT
ACTAAGAGAAGTCACAAAAGCAAGCGGAAGCACACTGAGAAACTTTG
TTTAGTTTTGAGGGTAGTACCTCAAAGAGCTAGTACATTGAAAACTG
AATATAATCCAAGCAAAAAACCGAGAAAATCAAAGAGAACAGATTGC
AAGGCGACCGAGAAGAGAATTCTTGAGTAAGGTCAAGTAGAAAAGG
GCGCACGGTGAATGCCTAGGCACTAAAAGCCGATGAAGGACGTGAC
GAACTACGAAAAGCTTCGGGGAGCGGTAAGTACGCAGTGATCCGGA
GATGTCCGAATGGGGGG (SEQ ID NO: 4)

In a preferred embodiment of the present invention, in the above combination, the lactic bacteria are in lyophilized form. This embodiment makes possible to administer a beneficial, high amount of the composition itself.

The combination according to the present invention can be administered also as it stands, but for practical purposes, it may be preferred to formulate the combination in a composition by admixing at least one excipient, which is acceptable and suitable for administration to a mammal, in particular a human or an animal, such as pets or production animals.

Examples of excipients are malt dextrin, microcrystalline cellulose, maize starch, rice starch, laevulose, lactose and dextrose.

The skilled reader will be able to use any suitable excipient according to the general knowledge. A guideline can be found in Remington's Pharmaceutical Sciences, latest edition, Mack Publishing and Co.

In another embodiment of the present invention, in the composition, said bacteria (a) and (b) are present in a concentration of from about 1x10⁸ to about 1x10¹² total bacteria per gram of composition.

In a further embodiment, the combination or composition of the present invention may comprise at least one of the following strains:
*Lactobacillus acidophilus, Lactobacillus buchneri, Lactobacillus brevis, Lactobacillus casei, Lactobacillus catenaforme, Lactobacillus cellobiosus, Lactobacillus crispatus, Lactobacillus curvatus, Lactobacillus delbrueckii, Lactobacillus jensenii, Lactobacillus leichmanii, Lactobacillus minutus, Lactobacillus plantarum Lactobacillus rogosae, Lactobacillus salivarius, Bifidobacterium adolescentis, Bifidobacterium angulatum, Bifidobacterium bifidum, Bifidobacterium catenulatum, Bifidobacterium dentium,* *Bifidobacterium eriksonii, Bifidobacterium infantis, Bifidobacterium longum, Bifidobacterium plantarum, Bifidobacterium pseudocatenulatum, Bifidobacterium pseudolongum, Streptococcus lactis, Streptococcus raffinolactis, Streptococcus thermophilus, Acidaminococcus fermenta, Cytophaga fermentans, Rhodoferax fermentans, Cellulomonas fermentans and Zymomonas mobilis.*

The combination, or the composition containing it, according to the present invention are able to exert their prophylactic and/or therapeutic activity in recovering the microbial equilibrium in the mammal body thanks to the particular combined presence of components (a) and (b).

A combination according to the present invention can also be provided by adding further strains of lactic acid bacteria or other microorganisms beneficial to the mammal. For example, the combination, or the composition containing it, further contains from about 85% to 5% by weight of one or more lactic acid bacteria, optionally in lyophilised form, selected from the group consisting of *bifidobacteria* and *Lactobacillus acidophilus.* These latter bacteria are present in an effective concentration, for example from about 1x10⁸ to about 1x10¹² total bacteria per gram of composition.

Although there are no limitations in the choice of the further beneficial bacteria which can optionally be added to the combination or composition according to the present invention, provided that they do not affect the activity, a preferred mixture of bifidobacteria comprises at least one of *Bifidobacterium lactis, Bifidobacterium bifidum, Bifidobacterium breve* and *Bifidobacterium longum.* The balance of the bifidobacteria mixture is not critical and can be determined by the person skilled in the art just resorting to the general knowledge. For sake of convenience, said bifidobacteria are in an approximately equal weight distribution.

The further lactic acid bacteria, which can be added to the above combination can be of any lactic acid bacteria strain, provided they do not affect its activity, or, preferably enhance or give a synergistic effect. As said above, preferred further strains are *bifidobacteria* or *Lactobacillus acidophilus.* The present invention can be carried out also with mutants or derivatives of said additional strains, provided that they have the same or better activities.

Preferably, lactic acid bacteria of the above additional strains having the following 16S-23S region genetic sequences are used.

### Lactobacillus acidophilus

CGGTTCAGCACGCCGCGGTGAATACGTTCCCGGGCCTTGTACACACC
GCCCGTCACACCATGGGAGTCTGCAATGCCCAAAGCCGGTGGCCTAA
CCTTCGGGAAGGAGCCGTCTAAGGCAGGGCAGATGACTGGGGTGAA
GTCGTAACAAGGTAGCCGTAGGAGAACCTGCGGCTGGATCACCTCCT
TTCTAAGGAAGCGAAGGATATGGAGAGTAGAAATACTAAGAGAAGTA
TCCAGAGCAAGCGGAAGCACACTAAGAAACTTTGTTTAGTTTTGAGG
GTAGTACCTCAAAAGAGTTAGTACATTGAAAACTGAATATAATCCAA
GCAAAAAACCGAGACAATCAAAGAGAACAGATTGTAGAGCGACCGA
GAAGAGAATTCTTGGGTAAGGTCAAGTAGAAAAGGGCGCACGGTGA
ATGCCTAGGCACTAACAGCCGATGAAGGACGTGACGAACTACGAAAA
GCTTCGGGGAGCGGTAAGTACGCAGTGATCCGGAGATGTCCGAATG
GGGGGACCCCAC (SEQ ID NO: 5)

### Bifidobacterium lactis

CGAAATCACCTCCTTTCTACGGACAATATGGGCGTGCATGGTTTTGT
GCACGGTTTTGTGGCTGGGGTTTGCCGAGTGCGATGGTTTGTCGTCG
CCGCCCTGGCTTGCTGGCGTGGAAGAGTCGTATTGGATTGTGTGGTG
TGGTGCAGTGCTGTTGGGTTCCCTGGCCGCCACCGGCCCCTGGTGTG

GGGTGTGGTTGCCAGTGTGTCCCCCAGGTCGGTTGCCCGGTGCCTGT
TGGTGCGGGGTGGGTGTCCTGGTGGGGTGGTGGTTTGAGAACTGGA
TAGTGGACGCGAGCATCGGCCCGCCCGTTGGGGTGGGGTTGGTGTT
CGTACTGTGATTCGGATCCGAAGGANATTGTTTTTCTTGGGTTCGT
TCGTANNNATAGTNNANNTGGGGGCCTGCCGTGTGTGGTNGCGTCC
CCGTGATCTAGAAATCGTTCTAGGCTTTTTCAGTAGTGCTTTGCTCAA
AACGGCCGTGTTGTGGTTGTCGTGAGGGCGTAAANCNTG(SEQ ID
NO: 6)

### Bifidobacterium breve

GAATCACCTCCTTTCTACGGAGAATGCAGCCCGGATGTTCGTCCGGC
GGTGCGAACCCCGACGCCGCGGCAGTCGCGGCGGTTTGGGGCTGCT
GGTGTGGAAGAGATCATGGGCTTTCGGTTCGTCCGTCGTGGGATGTT
CCGCGGGAGTGGTGCGGATGCGCTTTTGGGCTCCCGGATCGCCACC
CCAGGCTTTGGCCTGGCGCGATTCGATGCCCATCGTGCCTGATGGCG
GGCTGGTCCCGTCCGGACGGCGTGGTGGTGCGTGGTGGCTTGAGAA
CTGGATAGTGGACGCGAGCAAGACGGGGTTTCCTTTTGATTCCTCGT
TCTTGCTGTTGATTTCGAATCGAACTCTATTTTAGTGTTTGTTTCGA
TCGTTTTGTGATCATTTTAGTGTGATGATTTGTCGTCTGGGAATTTGC
TAGGAAGGATCCTTGCGGGCCATGCGCACCGTGTGGTGCGTGTTGC
CTGCAAGGGCGTATGGTGGATGCCTTGGCACCAAGAA (SEQ ID NO:
7)

### Bifidobacterium bifidum

AATCACCTCCTTTCTACTGAAATATGTGGCGNGCATGGTTTTGTGCA
CGGTTTTGTGGCTGGGGTTTGCCGAGTGCGATGGTTTGTCGTCGCCG
CCCTGGCTTGCTGGCGTGGAAGAGTCGTATTGGATTGTGTGGTGTGG
TGCGTGCTGTTGGGTTCCCTGGCCGCCACCGGCCCCTGGTGTGGGG
TGTGGTTGCCAGTGTGTCCCCCAGGTCGGTTGCCCGGTGCCTGTTGG
TGCGGGGTGGGTGTCCTGGTGGGGTGGTGGTTTGAGAACTGGATAG
TGGACGCGAGCATCGGCCCGCCCGTTGGGGTGGGGTTGGTGTTCGT

ACTGTGATTCGGATCCGAGGAATTGTTTTTCTTGGGTTCGTTCGTAA
TCGTATGGGGGCGTGCCGTGTGTGGTGCAGTCCCCGTGATCTGATGT
CGTTCTANGGTTTTTCAGTAATGCTTTGGTCATGGCGGCCGTGTTGT
GGTTGTCGTGATGGGCGTATGGNGGATGCCTTGGCACCAGA (SEQ ID
NO: 8)

The composition according to the present invention can take different forms, provided they are suitable for administration to a mammal. For example, they preferably take the form of a nutritional composition, for human administration, or the form of a fodder, for animal administration. These nutritional compositions or fodders are fit for administration in order to meet the prophylactic purpose of the combination according to the present invention.

Alternatively, in case of well established pathology, it may be preferable to formulate the combination according to the present invention in the form of a pharmaceutical composition. In fact, the combination herein disclosed can be used as a true medicament.

In this case, the expert in the art can also provide to add to the combination of the present invention a drug of choice for the pathology to be treated, for example anticholinergics, analgesics, adrenergics, antiiflammatories, hepatoprotective agents, antilipemics, antispastics, vitamins, antioxidants, unsaturated or saturated lipids and mixtures, hormones, antibodies, anti-TNF-α antibodies, cytokines or anticytokines, toxins or antitoxins, toxoids, anti-diabetes agents, 5-aminosalycilic acid, melatonin, prebiotics.

In a preferred embodiment of the present invention, the combination or composition are suitable for enteric or vaginal administration.

A preferred example of composition of the present invention comprises:
a) from about 40 to about 60% by weight of *Streptococcus termophilus;*
b) from about 7 to about 15% by weight of *Lactobacillus paracasei;*
c) from about 8 to about 10% by weight of *Lactobacillus pentosus;*
d) from about 7 to about 10% by weight of *Lactobacillus acidophilus;*
e) from about 8 to about 10% by weight of *Lactobacillus helveticus;*
f) from about 30 to about 50% by weight of a mixture of bifidobacteria and at least one excipient acceptable for human or veterinary use.

The correction or restore of bacteria flora can be achieved by administering the combination or composition thereof of the present invention in different forms, conveniently chosen by the skilled person depending on the purpose to be achieved. In case a prophylactic action is desired, the combination of the present invention can be formulated in a nutritional composition or included in food. For veterinary purposes, the combination is conveniently included in a fodder. In case a therapeutic action is to be achieved, the combination of the invention will take the form of a medicament.

Other than correcting and/or restoring imbalances of the intestinal flora in mammals, the combination of the present invention, in the form of a medicament or a nutritional composition, can be used for the treatment or prophylaxis of inflammatory conditions or a degenerative disease of the gastrointestinal tract, liver, pancreas, vagina, mouth, anus or any fistolous tract. Examples of inflammatory conditions or a degenerative disease or of degenerative or of functional disease of the gastrointestinal tract are irritable bowel syndrome, inflammatory bowel disease, ulcerative colitis, Crohn's disease, pouchitis, allergic colitis, eosinophilic colitis, actinic radiation colitis, diverticulitis, diverticulosis, constipation, food intolerance, e.g. gluten or fructose, and allergic disease of the gut, diarrhoea, hepatic encephalopathy, portal hypertension, bacterial peritonitis, hepatopathies, pancreatic diseases, diabetes, cancer, autoimmune disorders.

In another embodiment of the present invention, the combination or composition thereof of the present invention is useful for the preparation of a medicament, a nutritional composition, a food or a fodder capable of modulating the activity of the melatonin and melatonin-like receptors at the enteric and systemic level, modulating the motility of the gastrointestinal tract and/or reducing spasms and/or regulating circadian cycle.

In still another embodiment of the present invention, the combination or composition thereof of the present invention is useful for the preparation of a medicament, a nutritional composition, a food or a fodder capable of modulating the activity of COX-2.

In still another embodiment of the present invention, the combination or composition thereof of the present invention is useful for the preparation of a medicament, a nutritional composition, a food or a fodder capable of inactivating and/or digesting cytokines.

The compositions of the invention can be prepared with methods well-known to those skilled in dairy technology, enabling the presence of viable bacteria at concentrations ranging between 1x10⁸ and 1x10¹² bacteria per gram of the composition.

The compositions of the invention can be made in conventional pharmaceutical forms adapted for human or veterinary use, such as for example tablets, coated tablets, capsules, enteric coated tablets or capsules, packets, solutions, sachets, suspensions, emulsions, suppositories, pellets, syrups, vaginal suppositories, and are prepared in the usual manner by mixing active ingredients in the mentioned amounts, optionally adding excipients and/or carriers, adjuvants and/or dispersing agents. Water may be used as the diluent. Organic solvents can be used in the form of adjuvants. Adjuvants can be for example, nontoxic organic solvents such as paraffins, vegetable oils (peanut oil or sesame oil) glycerin, glycols (propylene glycol, polyethylene glycol), solid carriers such as for example natural mineral flours (kaolin, talc), synthetic mineral flours (silicates for example), sugar (cane sugar for example), emulsifiers (alkylsulfonates or arylsulfonates and the like), dispersants (lignin, methylcellulose, starch and polyvinylpyrrolidone, for example) and lubricants (magnesium stearate, talc, stearic acid, sodium laurylsulfonate, for example). Preferred excipients for the composition of the invention are maltodextrin, microcrystalline cellulose, maize starch, levulose, lactose and dextrose. Formulations for rectal administration may take the form of a suppository with the usual carriers such as cocoa butter, hard fat or polyethylene glycol and any suspension in any suitable liquid for enteric administration..

The administration takes place in the usual manner, preferably by the oral route. Orally administrable forms can contain, in addition to usual excipients, additives such as sodium citrate, calcium carbonate, calcium dihydrogen phosphate, together with several additional substances such as starch, gelatin and the like. Rectal administration may also be used. In case of liquid compositions compatible coloring agents or flavoring substances may be added.

As an optional component, the compositions may contain such compatible herbal extracts having useful healthy activity, chitosane and chitosane-like compounds, in amounts of from 1 to 20% by weight, based on the total weight of the composition. As mentioned above, to prepare the compositions, the individual microorganisms in the dehydrated form are mixed in appropriate proportions and the mixture is then mixed with the excipients or optionally with other pharmaceuticals.

The present invention is characterized by the administration of high doses, in the hundreds of billions, of protective lactic acid bacteria cells, calculated on a daily basis. Administration is typically divided into several doses over the day, usually three times a day, even though one single dose is equally effective and can be given to facilitate the compliance of the patient. As the weight of bacteria grown varies from batch to batch, for accuracy it is convenient to reckon the amounts administered on the basis of the number (actual or estimated) of bacteria cells administered per day. Daily doses may range from as low as about 100 x 10⁹ cells up to about 3,600 x 10⁹ or more, preferably at least 450 x 10⁹ cells and desirably within a "core" range of from about 900 x 10⁹ to about 1,800 x 10⁹ bacterial cells per day. The amount administered by the clinician may vary within the above ranges and will be dependent upon the patient's condition and response to therapy.

The dietary supplements used in the methods of the present invention may include in addition to the probiotic lactic acid bacteria one or more of plant extracts, vitamins, charcoal, minerals as well as flavoring, coloring or stabilizing ingredients.

Doses in these amounts quickly develop high concentrations of protective bacteria in the intestinal lumen and the feces. The dietary or pharmaceutical compositions used in the method of the present invention may be administered orally or rectally. Administration may be accompanied with other active ingredients used to treat the particular clinical indication being treated. Illustrative actives include corticosteroids, anti-TNFa antibodies, cytokines, 5ASA and its derivatives, immunosuppressants such as cyclosporine, and various antibodies and genetically modified bacteria among others as will be apparent to the skilled clinician.

A preferred formulation designed for oral administration is the lactic acid bacteria-containing composition in starch-coated pellets or associated in a pharmaceutical presentation with substances such as carbohydrates, sugars, lipids, physiologically acceptable polymers and copolymers and the like. These materials are used to protect the bacteria's viability after ingestion and to facilitate passage of the probiotic lactic acid bacteria composition through the stomach and into the intestine such that the probiotic lactic acid bacteria composition is released or otherwise made available at the appropriate site requiring treatment in the intestinal pathway.

A specific embodiment of the invention relates to patients who are irradiated in the pelvic area. In these patients a fairly mild side-effect is frequently noted, which is diarrhoea. In addition to having a negative effect on the quality of life of the patient subjected to x-ray therapy treatment, this condition may in extreme cases mean that the x-ray treatment itself has to be suspended. Therefore, the present invention can be useful to increase the amount of x-rays and/or chemotherapy to the patient.

As a rule, diarrhoea arises approximately two weeks after the start of the treatment and is correlated with the magnitude of the dose per fraction and with the volume exposed to radiation. The intestinal tract, which is most sensitive to x-rays, is composed of the epithelium of the small intestine. As a matter of fact, even at moderate doses of x-ray therapy, difficulty in absorbing fats and impermeability are noted. Within a few hours after the radiation is completed, the proliferative activity of the crypt ceases (in its middle third), and cellular necroses with pyknosis, karyolysis, karyorrhexis, and an accumulation of debris in the cryptic lumen are observed.

The initial reaction is of the precocious type and can occur during x-ray therapy. This is represented by an acute enteritic picture, whose severity is related to the portion of the small intestine that is irradiated and the dosages administered.

The enteritic process can become more complicated, with a severe loss of fluids and electrolytes; the x-ray-induced change in the function of the mucus-lymphoid barrier owing to the interfacial lymphocytolysis which depletes the Peyer's patches and to the exfoliation of the unreplaced enterocytes, may promote the development of fatal septicemic pictures.

The literature reports various dietetic and pharmacological means to be helpful in preventing the diarrhoeal syndrome that develops in patients exposed to x-ray therapy treatment in the pelvic region. Previously used medical treatments are based on the use of sucralfate, smectite, milk enzymes, and acetylsalicylic acid.

In addition to an appropriate dietetic regime, it has been found these patients may be successfully treated with a preparation containing a high concentration of lyophilized live bacteria that consist of microflora arranged in a qualitatively and quantitatively optimum way to ensure a re-balancing action of the intestinal flora in order to reduce the impact of the diarrhoea, thereby improving the patient's quality of life and drastically decreasing the risks of the above-mentioned x-ray therapy having to be suspended.

The following examples further illustrate the invention.

### Example 1

The gastrointestinal levels of melatonin (5-methoxy-N-acetyltriptamine) are at least 400 times more than in the pineal gland. Melatonin relaxes gastrointestinal muscles by specifically blocking nicotinic channels. Melatonin has a protective effect on the development of gastrointestinal ulcers, can prevent the development of colitis, and has oncostatic effects. Unfortunately, 92-97% of melatonin is degraded on a single passage through the liver, making the oral administration of the molecule useless to the above described desired effects.

Now, we have found that an effective way to supplement the host with melatonin is to administer species of lactic acid bacteria which contain melatonin. According to the following table, in our experiments, we found that the following strains were particularly suited for the scopes of the invention.

| Lactic acid bacteria | pg of melatonin/g of bacteria (10¹⁰ CFU) |
|---|---|
| *L. pentosus* | 12.16 |
| *L. paracasei* | 12.04 |
| *S. thermophilus* | 4.64 |
| *L. helveticus* | 1.02 |

The bacteria, in combination by two or more, can significantly colonize the intestinal tract and therefore be a permanent source of melatonin for the gastrointestinal tract of the person who ingested them.

### Example 2

Chemotherapy-induced diarrhoea (CID) is one of the most important problems in patients with cancer. The signs and symptoms include watery diarrhoea associated with cramping, fevers, leukopenia and ultimately vascular collapse.

We treated 32 cancer patients with diarrhoea secondary to fluoro-pyrimidine-based therapy. 16 patients received loperamide per os, 4 mg initially, then 2 mg bid for 4 days. Only in 4 patients the diarrhoea was resolved by the end of the treatment. 16 patients received a composition of (per packed):

| | |
|---|---|
| *S. thermophilus* | 5.71x10¹¹ |
| *L. paracasei* | 7.93x10¹⁰ |
| *L. pentosus* | 1.59x10¹⁰ |
| *L. acidophilus* | 3.18x10¹⁰ |
| *L. helveticus* | 1.59x10¹⁰ |
| *B. breve* | 1.42x10¹¹ |
| *B. lactis* | 2.12x10⁹ |
| *B. bifidum* | 1.42x10¹¹ |

two packets per day for 4 days. At the end of the period, the diarrhoea was resolved in 9 out of the 16 patients treated.

The same composition can be further strengthened by adding S. *fecium.* However, *S. fecium* is dangerous in seriously depressed patients (risk of sepsis) and therefore, should be added only to preparations made for non immunodepressed people.

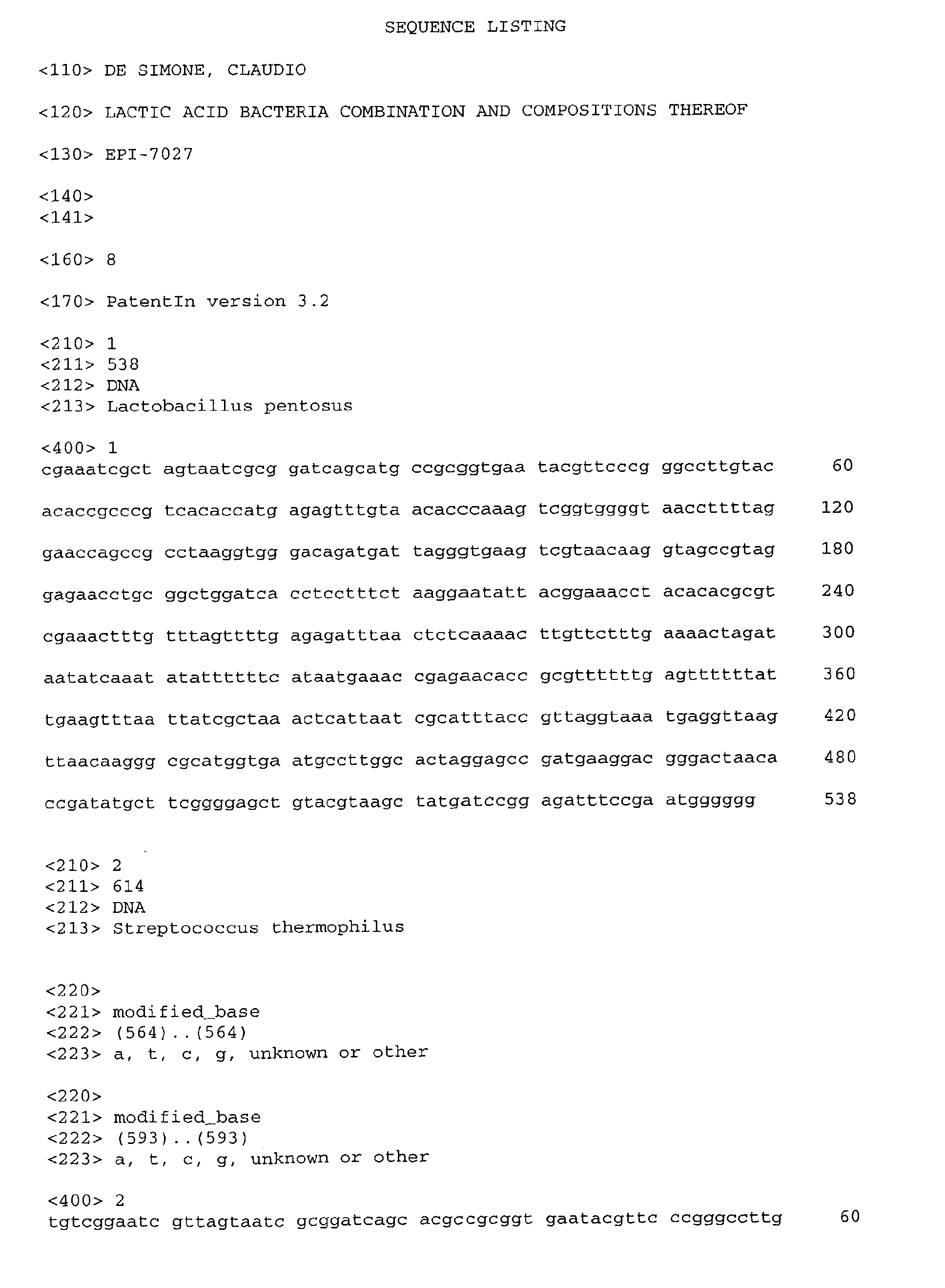

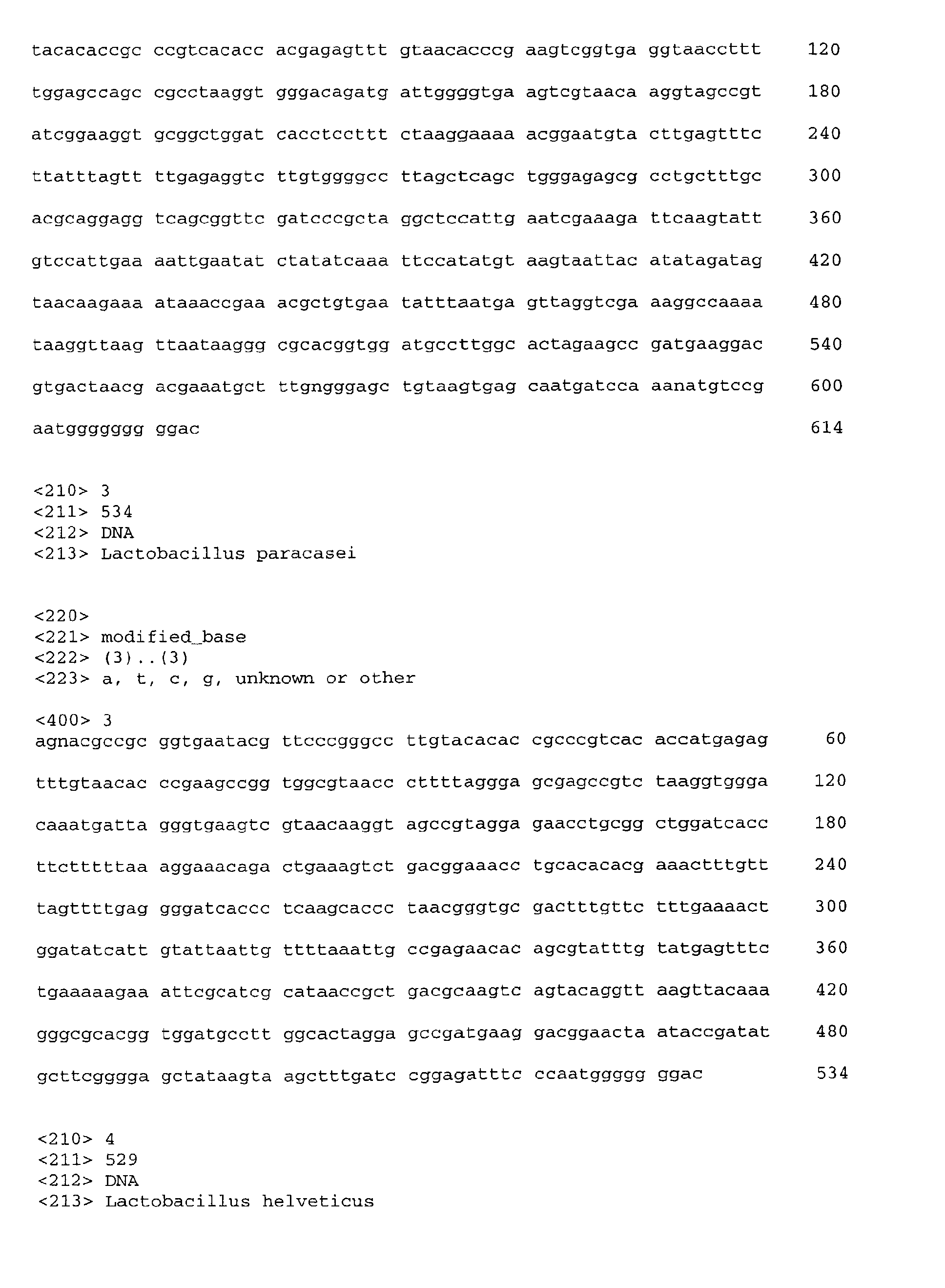

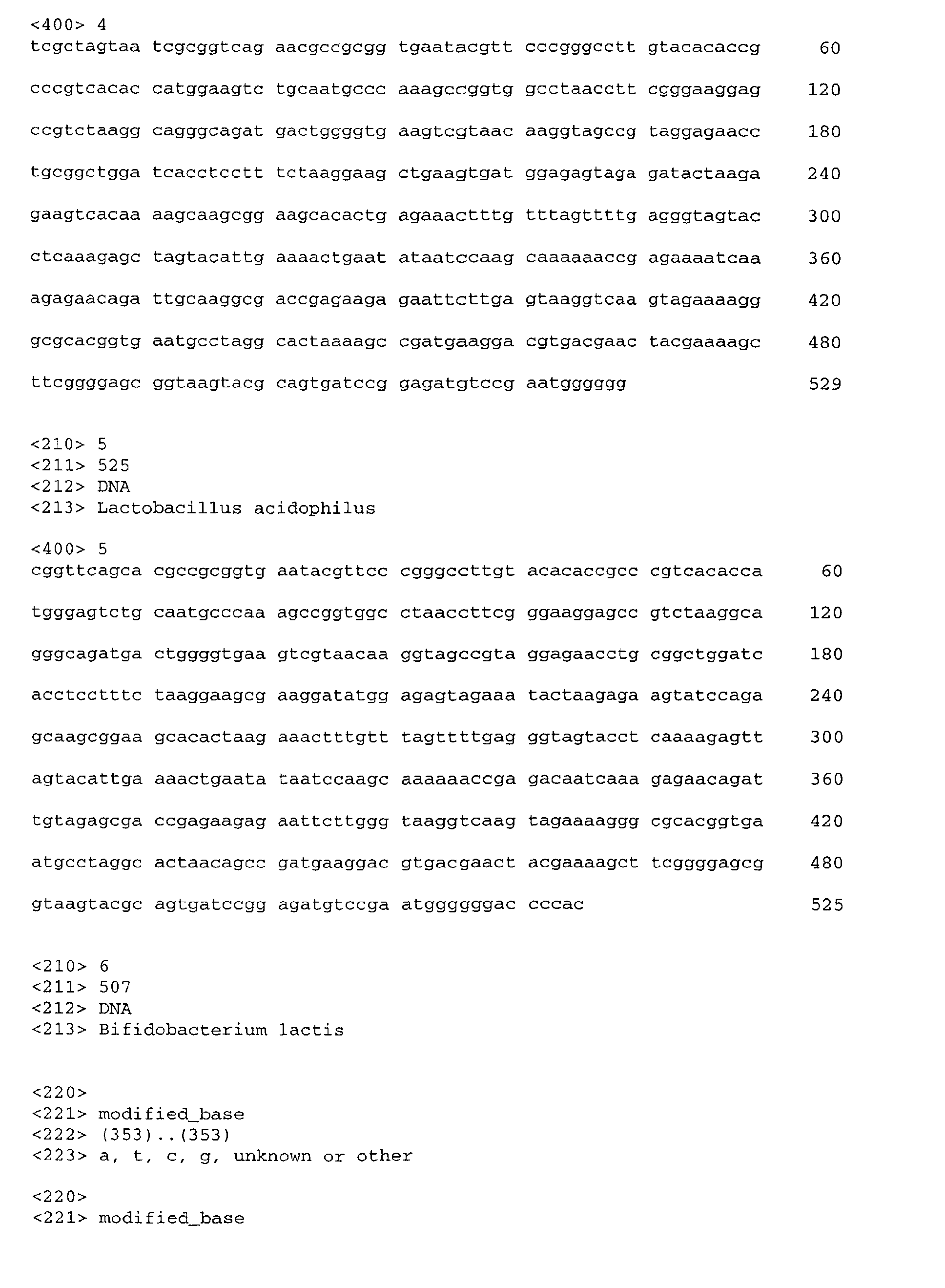

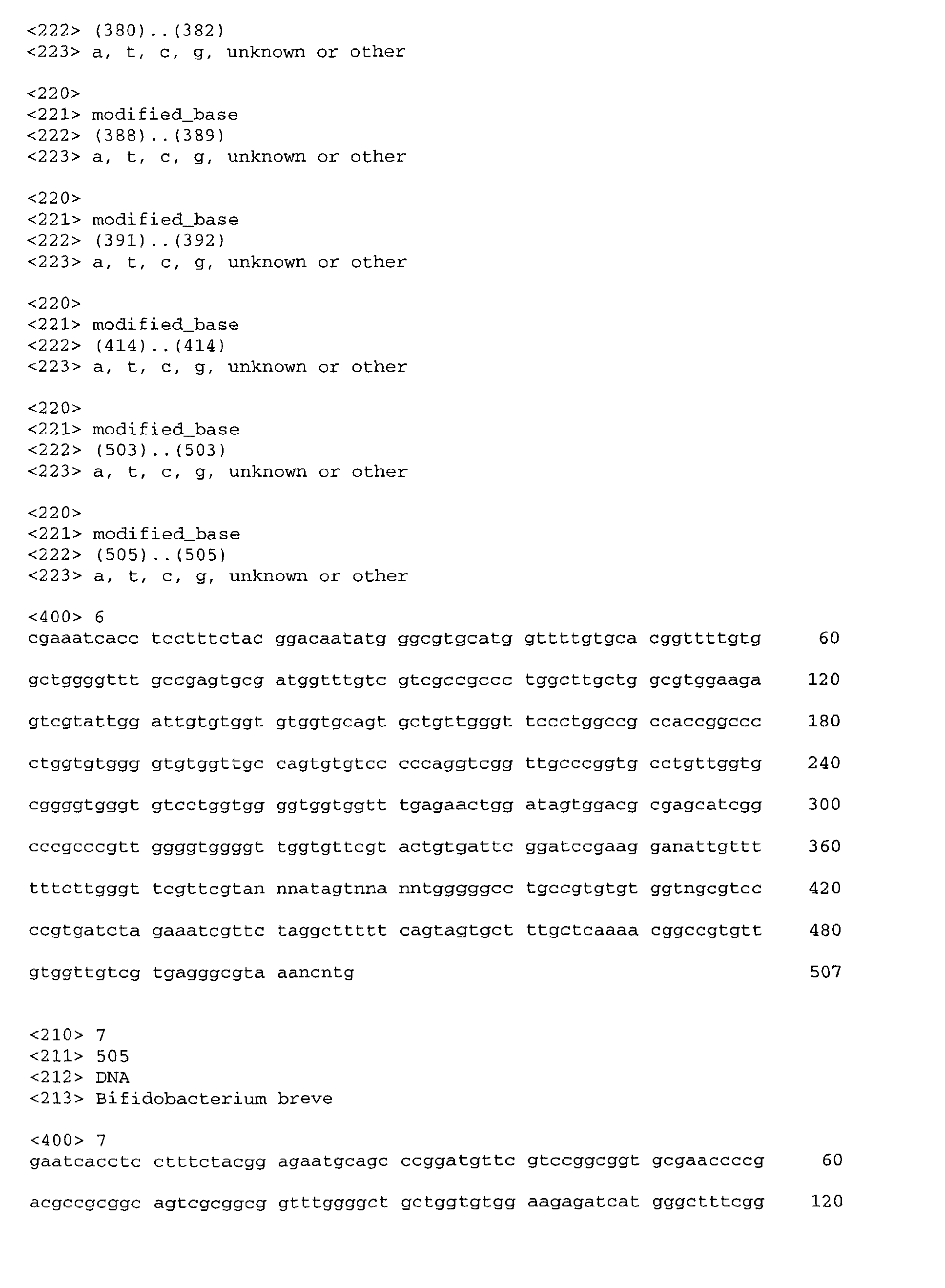

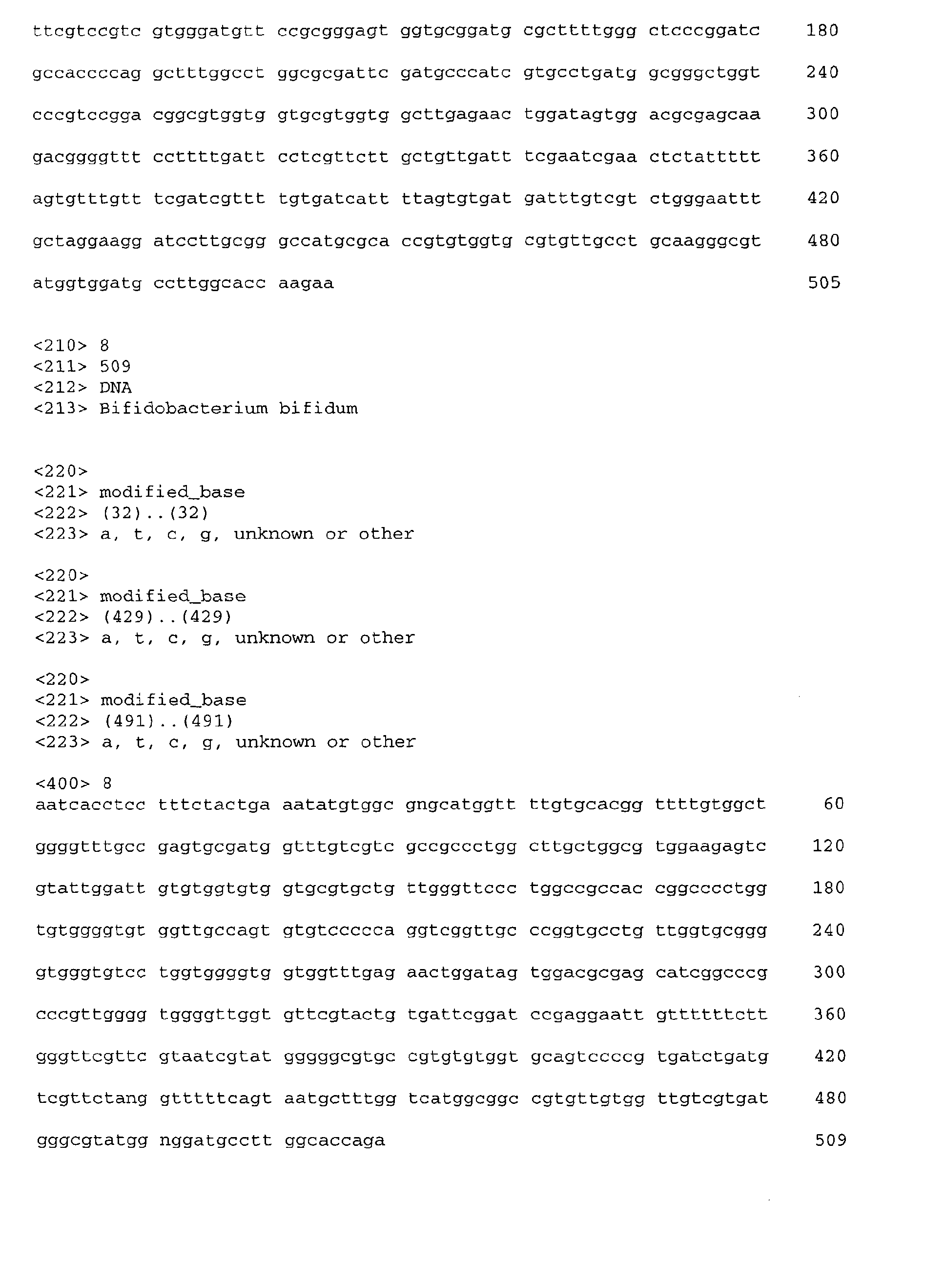

## Claims

1. Lactic acid bacteria combination consisting of:
(a) from about 5% to about 95% by weight of total combination of *Lactobacillus pentosus* and
(b) from about 5% to about 95% by weight of total combination of at least one strain of *Streptococcus thermophilus* and/or *Lactobacillus paracasei* and/or *Lactobacillus helveticus;* and/or mutants or derivatives of (a) and/or (b).

2. The combination according to Claim 1, wherein said bacteria have the following nucleotide sequences of the 16S-23S region:
*Lactobacillus pentosus* 
CGAAATCGCTAGTAATCGCGGATCAGCATGCCGCGGTGAATACGTTC
CCGGGCCTTGTACACACCGCCCGTCACACCATGAGAGTTTGTAACAC
CCAAAGTCGGTGGGGTAACCTTTTAGGAACCAGCCGCCTAAGGTGG
GACAGATGATTAGGGTGAAGTCGTAACAAGGTAGCCGTAGGAGAAC
CTGCGGCTGGATCACCTCCTTTCTAAGGAATATTACGGAAACCTACA
CACGCGTCGAAACTTTGTTTAGTTTTGAGAGATTTAACTCTCAAAACT
TGTTCTTTGAAAACTAGATAATATCAAATATATTTTTCATAATGAAA
CCGAGAACACCGCGTTTTTGAGTTTTTATTGAAGTTTAATTATCGC
TAAACTCATTAATCGCATTTACCGTTAGGTAAATGAGGTTAAGTTAAC
AAGGGCGCATGGTGAATGCCTTGGCACTAGGAGCCGATGAAGGACG
GGACTAACACCGATATGCTTCGGGGAGCTGTACGTAAGCTATGATCC
GGAGATTTCCGAATGGGGGG (SEQ ID NO: 1)
 *Streptococcus thermophilus* 
TGTCGGAATCGTTAGTAATCGCGGATCAGCACGCCGCGGTGAATACG
TTCCCGGGCCTTGTACACACCGCCCGTCACACCACGAGAGTTTGTAA
 
CACCCGAAGTCGGTGAGGTAACCTTTTGGAGCCAGCCGCCTAAGGTG
GGACAGATGATTGGGGTGAAGTCGTAACAAGGTAGCCGTATCGGAA
GGTGCGGCTGGATCACCTCCTTTCTAAGGAAAAACGGAATGTACTTG
AGTTTCTTATTTAGTTTTGAGAGGTCTTGTGGGGCCTTAGCTCAGCT
GGGAGAGCGCCTGCTTTGCACGCAGGAGGTCAGCGGTTCGATCCCG
CTAGGCTCCATTGAATCGAAAGATTCAAGTATTGTCCATTGAAAATT
GAATATCTATATCAAATTCCATATGTAAGTAATTACATATAGATAGTA
ACAAGAAAATAAACCGAAACGCTGTGAATATTTAATGAGTTAGGTCG
AAAGGCCAAAATAAGGTTAAGTTAATAAGGGCGCACGGTGGATGCCT
TGGCACTAGAAGCCGATGAAGGACGTGACTAACGACGAAATGCTTTG
NGGGAGCTGTAAGTGAGCAATGATCCAAANATGTCCGAATGGGGGG
GGGAC (SEQ ID NO: 2)
 *Lactobacillus paracasei* 
AGNACGCCGCGGTGAATACGTTCCCGGGCCTTGTACACACCGCCCGT
CACACCATGAGAGTTTGTAACACCCGAAGCCGGTGGCGTAACCCTTT
TAGGGAGCGAGCCGTCTAAGGTGGGACAAATGATTAGGGTGAAGTC
GTAACAAGGTAGCCGTAGGAGAACCTGCGGCTGGATCACCTTCTTTT
TAAAGGAAACAGACTGAAAGTCTGACGGAAACCTGCACACACGAAAC
TTTGTTTAGTTTTGAGGGGATCACCCTCAAGCACCCTAACGGGTGCG
ACTTTGTTCTTTGAAAACTGGATATCATTGTATTAATTGTTTTAAATT
GCCGAGAACACAGCGTATTTGTATGAGTTTCTGAAAAAGAAATTCGC
ATCGCATAACCGCTGACGCAAGTCAGTACAGGTTAAGTTACAAA
GGGCGCACGGTGGATGCCTTGGCACTAGGAGCCGATGAAGGACGGA
ACTAATACCGATATGCTTCGGGGAGCTATAAGTAAGCTTTGATCCGG
AGATTTCCCAATGGGGGGGAC (SEQ ID NO: 3)
 *Lactobacillus helveticus*

TCGCTAGTAATCGCGGTCAGAACGCCGCGGTGAATACGTTCCCGGGC
CTTGTACACACCGCCCGTCACACCATGGAAGTCTGCAATGCCCAAAG

CCGGTGGCCTAACCTTCGGGAAGGAGCCGTCTAAGGCAGGGCAGAT
GACTGGGGTGAAGTCGTAACAAGGTAGCCGTAGGAGAACCTGCGGC
TGGATCACCTCCTTTCTAAGGAAGCTGAAGTGATGGAGAGTAGAGAT
ACTAAGAGAAGTCACAAAAGCAAGCGGAAGCACACTGAGAAACTTTG
TTTAGTTTTGAGGGTAGTACCTCAAAGAGCTAGTACATTGAAAACTG
AATATAATCCAAGCAAAAAACCGAGAAAATCAAAGAGAACAGATTGC
AAGGCGACCGAGAAGAGAATTCTTGAGTAAGGTCAAGTAGAAAAGG
GCGCACGGTGAATGCCTAGGCACTAAAAGCCGATGAAGGACGTGAC
GAACTACGAAAAGCTTCGGGGAGCGGTAAGTACGCAGTGATCCGGA
GATGTCCGAATGGGGGG (SEQ ID NO: 4)

3. The combination according to Claim 1 or 2, in which said bacteria are in lyophilised form.

4. A composition comprising the combination of any one of claims 1-3, in admixture with at least one acceptable excipient.

5. The composition according to Claim 4, in which said bacteria (a) and (b) are present in a concentration of from about 1x10⁸ to about 1x10¹² total bacteria per gram of composition.

6. The combination according to any one of Claims 1-3 or the composition according to any one of Claims 4 or 5, which further contains from about 85% to 5% by weight of one or more lactic acid bacteria, optionally in lyophilised form, selected from the group consisting of *bifidobacteria, Lactobacillus acidophilus.*

7. The composition according to Claim 6, in which said bacteria are present in a concentration of from about 1x10⁸ to about 1x10¹² total bacteria per gram of composition.

8. The composition according to Claim 6 or 7, in which said bifidobacteria are a mixture of *Bifidobacterium lactis, Bifidobacterium bifidum* and *Bifidobacterium breve.*

9. Composition according to Claim 8, wherein said bacteria have the following nucleotide sequences of the 16S-23S region:
*Lactobacillus acidophilus* 
CGGTTCAGCACGCCGCGGTGAATACGTTCCCGGGCCTTGTACACACC
GCCCGTCACACCATGGGAGTCTGCAATGCCCAAAGCCGGTGGCCTAA
CCTTCGGGAAGGAGCCGTCTAAGGCAGGGCAGATGACTGGGGTGAA
GTCGTAACAAGGTAGCCGTAGGAGAACCTGCGGCTGGATCACCTCCT
TTCTAAGGAAGCGAAGGATATGGAGAGTAGAAATACTAAGAGAAGTA
TCCAGAGCAAGCGGAAGCACACTAAGAAACTTTGTTTAGTTTTGAGG
GTAGTACCTCAAAAGAGTTAGTACATTGAAAACTGAATATAATCCAA
GCAAAAAACCGAGACAATCAAAGAGAACAGATTGTAGAGCGACCGA
GAAGAGAATTCTTGGGTAAGGTCAAGTAGAAAAGGGCGCACGGTGA
ATGCCTAGGCACTAACAGCCGATGAAGGACGTGACGAACTACGAAAA
GCTTCGGGGAGCGGTAAGTACGCAGTGATCCGGAGATGTCCGAATG
GGGGGACCCCAC (SEQ ID NO: 5)
 *Bifidobacterium lactis* 
CGAAATCACCTCCTTTCTACGGACAATATGGGCGTGCATGGTTTTGT
GCACGGTTTTGTGGCTGGGGTTTGCCGAGTGCGATGGTTTGTCGTCG
CCGCCCTGGCTTGCTGGCGTGGAAGAGTCGTATTGGATTGTGTGGTG
TGGTGCAGTGCTGTTGGGTTCCCTGGCCGCCACCGGCCCCTGGTGTG
GGGTGTGGTTGCCAGTGTGTCCCCCAGGTCGGTTGCCCGGTGCCTGT
TGGTGCGGGGTGGGTGTCCTGGTGGGGTGGTGGTTTGAGAACTGGA
TAGTGGACGCGAGCATCGGCCCGCCCGTTGGGGTGGGGTTGGTGTT
CGTACTGTGATTCGGATCCGAAGGANATTGTTTTTCTTGGGTTCGT
 
TCGTANNNATAGTNNANNTGGGGGCCTGCCGTGTGTGGTNGCGTCC
CCGTGATCTAGAAATCGTTCTAGGCTTTTTCAGTAGTGCTTTGCTCAA
AACGGCCGTGTTGTGGTTGTCGTGAGGGCGTAAANCNTG (SEQ ID
NO: 6)
 *Bifidobacterium breve* 
GAATCACCTCCTTTCTACGGAGAATGCAGCCCGGATGTTCGTCCGGC
GGTGCGAACCCCGACGCCGCGGCAGTCGCGGCGGTTTGGGGCTGCT
GGTGTGGAAGAGATCATGGGCTTTCGGTTCGTCCGTCGTGGGATGTT
CCGCGGGAGTGGTGCGGATGCGCTTTTGGGCTCCCGGATCGCCACC
CCAGGCTTTGGCCTGGCGCGATTCGATGCCCATCGTGCCTGATGGCG
GGCTGGTCCCGTCCGGACGGCGTGGTGGTGCGTGGTGGCTTGAGAA
CTGGATAGTGGACGCGAGCAAGACGGGGTTTCCTTTTGATTCCTCGT
TCTTGCTGTTGATTTCGAATCGAACTCTATTTTTAGTGTTTGTTTCGA
TCGTTTTGTGATCATTTTAGTGTGATGATTTGTCGTCTGGGAATTTGC
TAGGAAGGATCCTTGCGGGCCATGCGCACCGTGTGGTGCGTGTTGC
CTGCAAGGGCGTATGGTGGATGCCTTGGCACCAAGAA (SEQ ID NO:
7)
 *Bifidobacterium bifidum*

AATCACCTCCTTTCTACTGAAATATGTGGCGNGCATGGTTTTGTGCA
CGGTTTTGTGGCTGGGGTTTGCCGAGTGCGATGGTTTGTCGTCGCCG
CCCTGGCTTGCTGGCGTGGAAGAGTCGTATTGGATTGTGTGGTGTGG
TGCGTGCTGTTGGGTTCCCTGGCCGCCACCGGCCCCTGGTGTGGGG
TGTGGTTGCCAGTGTGTCCCCCAGGTCGGTTGCCCGGTGCCTGTTGG
TGCGGGGTGGGTGTCCTGGTGGGGTGGTGGTTTGAGAACTGGATAG
TGGACGCGAGCATCGGCCCGCCCGTTGGGGTGGGGTTGGTGTTCGT
ACTGTGATTCGGATCCGAGGAATTGTTTTTCTTGGGTTCGTTCGTAA
TCGTATGGGGGCGTGCCGTGTGTGGTGCAGTCCCCGTGATCTGATGT
CGTTCTANGGTTTTTCAGTAATGCTTTGGTCATGGCGGCCGTGTTGT

GGTTGTCGTGATGGGCGTATGGNGGATGCCTTGGCACCAGA (SEQ ID
NO: 8)

10. The composition according to any one of Claims 6-9, in which said mixture contains said bifidobacteria in approximately equal weight distribution.

11. The composition of any one of Claims 4-10, which further contains *S. fecium.*

12. The composition of any one of Claims 4-11 in the form of a pharmaceutical composition or a nutritional composition or a food or a fodder.

13. The composition according to Claim 12, in which an excipient acceptable for human or veterinary use is present.

14. The composition according to Claim 13, in which said excipient is selected from the group consisting of maltodextrin, microcrystalline cellulose, maize starch, rice starch, levulose, lactose and dextrose.

15. The pharmaceutical composition of any one of Claims 12-14, which further comprises an effective dose of a drug.

16. The pharmaceutical composition of Claim 15, wherein said drug is selected from the group consisting of anticholinergics, analgesics, adrenergics, antiiflammatories, hepatoprotective agents, antilipemics, antispastics, vitamins, antioxidants, unsaturated or saturated lipids and mixtures, hormones, antibodies, anti-TNF-α antibodies, cytokines or anticytokines, toxins or antitoxins, toxoids, anti-diabetes agents, 5-aminosalycilic acid, melatonin, prebiotics.

17. The composition according to any one of claims 4-16, suitable for enteric or vaginal administration.

18. The composition of any one of claims 4-17, comprising:
a) from about 40 to about 60% by weight of *Streptococcus termophilus;*
b) from about 7 to about 15% by weight of *Lactobacillus paracasei;*
c) from about 8 to about 10% by weight of *Lactobacillus pentosus;*
d) from about 7 to about 10% by weight of *Lactobacillus acidophilus;*
e) from about 8 to about 10% by weight of *Lactobacillus helveticus;*
f) from about 30 to about 50% by weight of a mixture of bifidobacteria and at least one excipient acceptable for human or veterinary use.

19. The composition according to any one of claims 4-18 further comprising at least one of the following strains:
*Lactobacillus acidophilus, Lactobacillus buchneri, Lactobacillus brevis, Lactobacillus casei, Lactobacillus catenaforme, Lactobacillus cellobiosus, Lactobacillus crispatus, Lactobacillus curvatus, Lactobacillus delbrueckii, Lactobacillus jensenii, Lactobacillus leichmanii, Lactobacillus minutus, Lactobacillus plantarum Lactobacillus rogosae, Lactobacillus salivarius, Bifidobacterium adolescentis, Bifidobacterium angulatum, Bifidobacterium bifidum, Bifidobacterium catenulatum, Bifidobacterium dentium, Bifidobacterium eriksonii, Bifidobacterium infantis, Bifidobacterium longum, Bifidobacterium plantarum, Bifidobacterium pseudo-catenulatum, Bifidobacterium pseudolongum, Streptococcus lactis, Streptococcus raffinolactis, Streptococcus thermophilus, Acidaminococcus fermenta, Cytophaga fermentans, Rhodoferax fermentans, Cellulomonas fermentans and Zymomonas mobilis.*

20. The composition according to any one of claims 4-19 further comprising one or more of herbal extracts, chitosane and chitosane-like compounds.

21. Use of the combination of any one of Claims 1-3 for the preparation of a probiotic product to correct imbalances and/or restore bacteria flora to the gut.

22. The use according to Claim 21, wherein said product is in the form of a nutritional composition, a food or a fodder.

23. The combination of any one of Claims 1-3 for use as a medicament.

24. Use of the combination of any one of Claims 1-3 for the preparation of a medicament for treating an inflammatory condition or a degenerative disease of the gastrointestinal tract, liver, pancreas, vagina, mouth, anus or any fistolous tract.

25. The use of according to Claim 24, wherein said condition is selected from the group consisting of irritable bowel syndrome, inflammatory bowel disease, ulcerative colitis, Crohn's disease, pouchitis, allergic colitis, eosinophilic colitis, actinic radiation colitis, diverticulitis, diverticulosis, constipation, food intolerance and allergic disease of the gut, diarrhoea, hepatic encephalopathy, portal hypertension, bacterial peritonitis, hepatopathies, pancreatic diseases, diabetes, cancer, autoimmune disorders.

26. Use of the combination of any one of Claims 1-3 for the preparation of a medicament and or nutritional composition capable of modulating the activity of the melatonin and melatonin-like receptors at the enteric and systemic level, modulating the motility of the gastrointestinal tract and/or reduce spasms, regulating circadian cycle.

27. Use of the combination of any one of Claims 1-3 for the preparation of a medicament, a nutritional composition, a food or a fodder capable of modulating the activity of COX-2.

28. Use of the combination of any one of Claims 1-3 for the preparation of a medicament, a nutritional composition, a food or a fodder capable of inactivating and/or digesting cytokines.
